# EUROPEAN PATENT APPLICATION

(11) **EP 3 379 481 A1**
(43) Date of publication of application: **26.09.2018**
(21) Application number: 17186141.2
(22) Date of filing: 14.08.2017
(51) Int. Cl.: G06Q 30/02, G06Q 30/06, G06Q 50/22

(54) **SKIN PRODUCT FITTING METHOD AND ELECTRONIC APPARATUS THEREFOR**

(30) Priority: 23.03.2017 CN 201710177093
(71) Applicant: Cal-Comp Big Data, Inc., New Taipei City 22201 (TW)
(72) Inventor: PAI, Nai-Chiang, 22201 Shenkeng Dist., New Taipei City (TW); TANG, Ching-Yi, 22201 Shenkeng Dist., New Taipei City (TW)
(74) Representative: 2K Patentanwälte Blasberg Kewitz & Reichel

(57) **Abstract**

A skin product fitting method and an electronic apparatus therefor are provided. The method includes following steps: capturing a skin image of a current user by using an image capturing device and analyzing the skin image to determine a skin concern; obtaining a skin type of the current user; and sorting a plurality of skin products stored in a product database at least according to the skin concern and the skin type so as to provide product information fitting a skin condition of the current user.

## Description

### BACKGROUND

### Field of the Invention

The invention relates to a skin detecting technique and more particularly, to a skin product fitting method and an electronic apparatus therefor.

### Description of Related Art

Traditional skin detection usually adopts a professional skin analyzer to scan human skin in order to obtain its skin condition. A salesperson of a service provider can also recommend the corresponding skin care products according to the skin condition for product promotion. However, it is difficult for users to own such skin analyzer as the skin analyzer is usually expensive and extremely bulky. Meanwhile, the skin analyzer is equipped with microscopes with different magnifications to scan the skin and thus, can scan only a small part of skin area each time, which takes a long time for the operation. Moreover, the users must be well-trained in order to determine the skin condition by using the skin analyzer. Therefore, for the users who wish to know about their own skin conditions and determine whether their skin can be indeed improved by the skin products at all times, the skin analyzer may not satisfy their actual demands.

On the other hand, most of the users, expecting for doing the skin care in an efficient and time-saving manner, would like to learn care information regarding how to take care of the skin and improve the skin condition, how long it would take for the skin care, which skin product to use and so on. However, it is difficult for each person to effectively confirm whether the skin care products are suitable through a corresponding method as each person has different skin conditions, growth environments and diet habits. Thus, how to find out products most suitable for the user's current skin condition from a variety of skin care products has become one of the most wanted information when the user chooses the skin products.

### SUMMARY

It is an object of the present invention to provide an enhanced skin fitting method, assisting a user and enabling finding suitable skin fit products in a more convenience, reliable, economic and objective manner. It is a further object of the present invention to provide a corresponding electronic apparatus suited to perform such a method, having a cost-effective configuration.

These problems are solved by a skin product fitting method and an electronic apparatus therefore, as claimed by claim 1 and 10, respectively. Further advantageous embodiments are the subject-matter of the dependent claims.

The invention provides a skin product fitting method and an apparatus therefor which can provide product information fitting a user's current skin condition by detecting the user's skin condition and referencing information of the user, such as a skin type, product preferences, a life profile and so on.

A skin product fitting method of the invention is applicable to an electronic apparatus including an image capturing device. The method includes: capturing a skin image of a current user by using the image capturing device and analyzing the skin image to determine a skin concern; obtaining a skin type of the current user; and sorting a plurality of skin products stored in a product database at least according to the skin concern and the skin type so as to provide product information fitting a skin condition of the current user.

According to a further embodiment of the invention, the method further includes receiving the current user's operation of selecting from a plurality of product preferences to determine the product preferences of the current user; receiving the current user's life information to determine a life profile of the current user; and sorting the skin products according to the skin concern, the skin type, the product preferences and the life profile so as to provide the product information fitting the skin condition of the current user.

According to a further embodiment of the invention, the step of sorting the skin products according to the skin concern, the skin type, the product preferences and the life profile so as to provide the product information fitting the skin condition of the current user includes selecting multiple skin products that simultaneously meet the skin concern, the skin type, the product preferences and the life profile from the skin products as a first group and sorting the skin products in the first group; selecting multiple skin products that only meet the skin concern, the skin type and the product preferences from the skin products as a second group and sorting the skin products in the second group; selecting multiple skin products that only meet the skin concern, the skin type and the life profile from the skin products as a third group and sorting the skin products in the third group; selecting multiple skin products that only meet the skin concern and the skin type from the skin products as a fourth group and sorting the skin products in the fourth group; and providing the sorted skin products of the first group, the second group, the third group and the fourth group in order as the product information fitting the skin condition of the current user.

According to a further embodiment of the invention, the step of receiving the current user's life information to determine the life profile of the current user includes providing a plurality of options regarding the current user's living habits, wherein each of the options corresponds to at least one of a plurality of skin concerns; and receiving the current user's operation of selecting from the options, calculating a number of times of each of the skin concerns being corresponded to by the options selected by the current user as the life profile, wherein the number of times of each skin concern is employed to weight the skin products meeting the skin concern when the skin products are sorted.

According to a further embodiment of the invention, the step of analyzing the skin image to determine the skin concern includes recognizing a plurality of red spots in the skin image and calculating a ratio of the red spots to the skin image; comparing the ratio with an upper limit and a lower limit so as to select one of a plurality of expected skin goals as an expected skin goal of the current user, wherein the expected skin goals include healthiness, clarity, firmness, texture and brightness; if the ratio is greater than the upper limit, prompting the current user to see a doctor; if the ratio is between the upper limit and the lower limit, selecting the healthiness as the expected skin goal of the current user; and if the ratio is less than the lower limit, calculating a score of the skin image under each of the expected skin goals and selecting the expected skin goal having the lowest score as the expected skin goal of the current user.

According to a further embodiment of the invention, the step of analyzing the skin image to determine the skin concern when the healthiness is selected as the expected skin goal of the current user further includes determining whether the red spots cause pain and itching; if the red spots cause pain and itching, obtaining a median between the upper limit and the lower limit and comparing the ratio with the upper limit, the lower limit and the median; if the ratio is between the upper limit and the median, determining the red spots as the skin concern; and if the ratio is between the median and the lower limit, determining the red spots and repair as the skin concern.

According to a further embodiment of the invention, the step of analyzing the skin image to determine the skin concern when the healthiness is selected as the expected skin goal of the current user further includes: if it is determined that the ratio of the red spots is less than the lower limit, or the red spots do not cause pain and itching, calculating an amount of the red spots and comparing the amount with a preset amount; if the amount is less than the preset amount, determining whether the red spots cause pain, determining anti-acne as the skin concern if the red spots cause pain and determining moisturizing as the skin concern if the red spots do not cause pain; and if the amount is greater than or equal to the preset amount, determining whether the red spots cause pain and itching, prompting the current user to see a doctor if the red spots cause pain and itching and determining anti-acne as the skin concern if the red spots do not cause pain and itching.

According to a further embodiment of the invention, before the step of analyzing the skin image to determine the skin concern, the method further includes receiving the current user's operation of selecting from a plurality of expected skin goals and determining the skin concern according to the selected expected skin goal, wherein the expected skin goals comprise healthiness, clarity, firmness, texture and brightness.

According to a further embodiment of the invention, when the clarity is selected as the expected skin goal of the current user, the step of analyzing the skin image to determine the skin concern includes recognizing a plurality of dark spots in the skin image, calculating a ratio of the dark spots to the skin image and comparing the ratio with an upper limit and a lower limit; if the ratio is greater than the upper limit, prompting the current user to see a doctor; if the ratio if the ratio is between the upper limit and the lower limit, determining dark spots and whitening as the skin concern; and if the ratio is less than the lower limit, determining moisturizing as the skin concern.

According to a further embodiment of the invention, when the clarity is selected as the expected skin goal of the current user, the step of analyzing the skin image to determine the skin concern includes determining whether a difference between a grayscale value of a cheek zone and a grayscale value of an under-eye zone in the skin image is greater than or equal to a preset value; if the difference is greater than or equal to the preset value, prompting the current user to see a doctor; and if the difference is less than the preset value, determining dark circles as the skin concern.

According to a further embodiment of the invention, when the firmness is selected as the expected skin goal of the current user, the step of analyzing the skin image to determine the skin concern includes obtaining the skin type of the current user; if the obtained skin type is an oily type or a combination type, determining oil control as the skin concern; and if the obtained skin type is a dry type or a normal type, determining pore firming as the skin concern.

According to a further embodiment of the invention, when the texture is selected as the expected skin goal of the current user, the step of analyzing the skin image to determine the skin concern includes recognizing wrinkles, fine lines and pores to the skin image, calculating a ratio of a total area of the wrinkles, the fine lines and the pores in the skin image and comparing the ratio with an upper limit and a lower limit; if the ratio is less than the lower limit, determining whether an age of the current user is greater than or equal to a preset age, determining skin firmness as the skin concern if the age is greater than or equal to the preset age and determining moisturizing as the skin concern if the age is less than the preset age; if the ratio is between the upper limit and the lower limit, determining whether the current user cares about skin firmness, determining skin firming and anti-wrinkles as the skin concern if the current user does and determining anti-aging and anti-wrinkles as the skin concern if the current user does not; and if the ratio is greater than the upper limit, determining anti-aging and anti-wrinkles as the skin concern.

According to a further embodiment of the invention, when the brightness is selected as the expected skin goal of the current user, the step of analyzing the skin image to determine the skin concern includes determining whether a difference between a grayscale value of a left-cheek zone and a grayscale value of a right-cheek zone in the skin image is greater than or equal to a preset value; if the difference is greater than or equal to the preset value, determining whether the current user cares about partially uneven skin tone, determining uneven skin tone as the skin concern if the current user does and determining whitening as the skin concern if the current user does not; and if the difference is less than the preset value, determining moisturizing as the skin concern.

An electronic apparatus of the invention includes an image capturing device, a storage device and a processor. The image capturing device is configured for capturing a skin image of a current user. The storage device is configured for storing a plurality of modules. The processor is coupled to the image capturing device and the storage device and configured for accessing and executing the modules stored in the storage device. The modules include a skin concern determining module, a skin type obtaining module and a product information providing module. The skin concern determining module is configured for analyzing the skin image to determine a skin concern. The skin type obtaining module is configured for obtaining a skin type of the current user. The product information providing module is configured for sorting a plurality of skin products stored in a product database at least according to the skin concern and the skin type so as to provide product information fitting a skin condition of the current user.

According to a further embodiment of the invention, the modules further include a product preference determining module and a life profile determining module. The product preference determining module is configured for receiving the current user's operation of selecting from a plurality of product preferences to determine the product preferences of the current user. The life profile determining module is configured for receiving the current user's life information to determine a life profile of the current user. The product information providing module sorts the skin products according to the skin concern, the skin type, the product preferences and the life profile so as to provide the product information fitting the skin condition of the current user.

According to a further embodiment of the invention, the product information providing module includes selecting multiple skin products that simultaneously meet the skin concern, the skin type, the product preferences and the life profile from the skin products as a first group and sorting the skin products in the first group; selecting multiple skin products that only meet the skin concern, the skin type and the product preferences from the skin products as a second group and sorting the skin products in the second group; selecting multiple skin products that only meet the skin concern, the skin type and the life profile from the skin products as a third group and sorting the skin products in the third group; selecting multiple skin products that only meet the skin concern and the skin type from the skin products as a fourth group and sorting the skin products in the fourth group; and providing the sorted skin products of the first group, the second group, the third group and the fourth group in order as the product information fitting the skin condition of the current user.

According to a further embodiment of the invention, the life profile determining module includes providing a plurality of options regarding the current user's living habits, wherein each of the options corresponds to at least one of a plurality of skin concerns; and receiving the current user's operation of selecting from the options, calculating a number of times of each of the skin concerns being corresponded to by the options selected by the current user as the life profile, wherein the number of times of each skin concern is employed to weight the skin products meeting the skin concern when the skin products are sorted.

According to a further embodiment of the invention, the modules further include a skin condition detecting module configured for recognizing a plurality of red spots in the skin image and calculating a ratio of the red spots to the skin image, and comparing the ratio with an upper limit and a lower limit so as to select one of a plurality of expected skin goals as an expected skin goal of the current user, wherein the expected skin goals comprise healthiness, clarity, firmness, texture and brightness. If the ratio is greater than the upper limit, the current user is prompted to see a doctor. If the ratio is between the upper limit and the lower limit, the healthiness is selected as the expected skin goal of the current user. If the ratio is less than the lower limit, a score of the skin image under each of the expected skin goals is calculated, and the expected skin goal having the lowest score is selected as the expected skin goal of the current user.

According to a further embodiment of the invention, the skin concern determining module further determines whether the red spots cause pain and itching. If the red spots cause pain and itching, a median between the upper limit and the lower limit is obtained, and the ratio is compared with the upper limit, the lower limit and the median. If the ratio is between the upper limit and the median, the red spots are determined as the skin concern. If the ratio is between the median and the lower limit, the red spots and repair are determined as the skin concern.

According to a further embodiment of the invention, the modules further include an expected skin goal selecting module configured for receiving the current user's operation of selecting from a plurality of expected skin goals and determining the skin concern according to the selected expected skin goal, wherein the expected skin goals include healthiness, clarity, firmness, texture and brightness.

Based on the above, in the skin product fitting method and the apparatus therefor of the embodiments of the invention, an image capturing device of the electronic apparatus is configured for detecting the user's skin so as to obtain information of the user, including the skin type, the product preferences, the life profile and so on. Lastly, the information is integrated to sort the skin products so as to provide the product information fitting the user's skin condition.

In order to make the aforementioned and other features and advantages of the invention more comprehensible, several embodiments accompanied with figures are described in detail below.

### BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.
FIG. 1 is a schematic diagram illustrating an electronic apparatus according to an embodiment of the invention.
FIG. 2 is a flowchart illustrating a skin product fitting method according to an embodiment of the invention.
FIG. 3 is a flowchart illustrating a skin product fitting method according to an embodiment of the invention.
FIG. 4 is a flowchart illustrating a method of detecting the skin condition according to an embodiment of the invention.
FIG. 5 illustrates a process of determining the skin concern when healthiness is selected as the expected skin goal according to an embodiment of the invention.
FIG. 6 illustrates a process of determining the skin concern when clarity is selected as the expected skin goal according to an embodiment of the invention.
FIG. 7 illustrates a process of determining the skin concern when firmness is selected as the expected skin goal according to an embodiment of the invention.
FIG. 8 illustrates a process of determining the skin concern when texture is selected as the expected skin goal according to an embodiment of the invention.
FIG. 9 illustrates a process of determining the skin concern when brightness is selected as the expected skin goal according to an embodiment of the invention.

### DESCRIPTION OF EMBODIMENTS

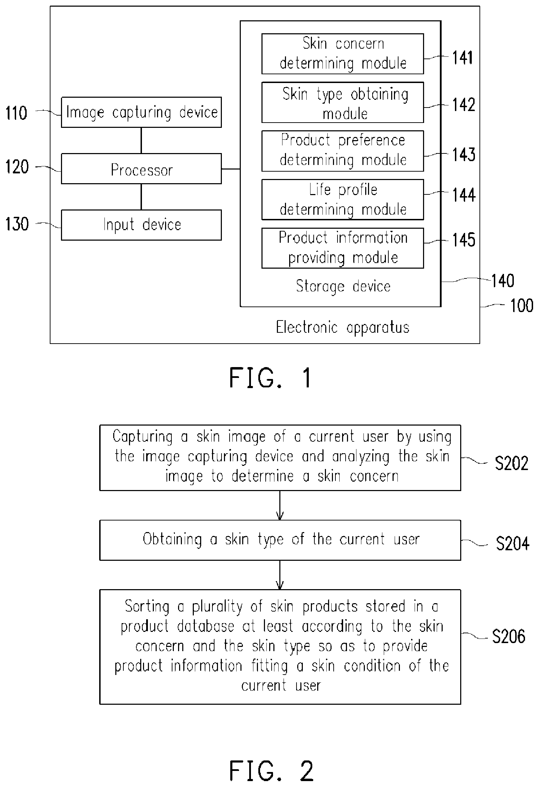

FIG. 1 is a schematic diagram illustrating an electronic apparatus according to an embodiment of the invention. Referring to FIG. 1, an electronic apparatus 100 of the present embodiment includes an image capturing device 110, a processor 120, an input device 130 and a storage device 140. The processor 120 is coupled to the image capturing device 110, the input device 130 and the storage device 140. The electronic apparatus 100 of the embodiment of the invention may be disposed on a mirror of a dressing table to capture a skin image of a user while the user looks in the mirror and provide product information fitting a skin condition of the user by using a display (not shown) disposed behind the mirror. It should be mentioned that in other embodiments, the electronic apparatus 100 may also be an electronic product, e.g., a smart cell phone, a tablet computer or a desktop computer, or a portable mirror case combined with a portable mirror.

The image capturing device 110 may be a camera equipped with a charge coupled device (CCD), a complementary metal-oxide semiconductor (CMOS) device or any other type of photosensitive device and is configured for capturing a skin image, especially a face skin image, of a current user.

The processor 120 may be a central processing unit (CPU), a microprocessor, a digital signal processor, a programmable controller, an application specific integrated circuit (ASIC), a programmable logic device (PLD) or any other device with data computing capability.

The input device 130 is a device capable of receiving user's operations, such as a mouse, a keyboard, a joystick or a touch panel. The input device 130 may also be a resistive, a capacitive, an optical or any other type touch sensing device which may be integrated with the display of the electronic apparatus 100 as a touch screen for receiving the user's touch operations. The glass of the touch screen may be made of a mirror material for having a mirror effect.

The storage device 140 may be any fixed or movable random access memory (RAM), read-only memory (ROM), flash memory or a like element or a combination of the aforementioned elements. In the present embodiment, the storage device 140 is configured to record a skin concern determining module 141, a skin type obtaining module 142, a product preference determining module 143, a life profile determining module 144 and a product information providing module 145. In other embodiments, the storage device 140 may also be configured to store a skin product database for the electronic apparatus 100 to search so as to provide product information. The modules are, for example, computer programs stored in the storage device 140 and may be loaded into the processor 120, such that the processor 120 can execute functions of a skin product fitting method of the application. The modules may be all integrated in an application program (APP), or partially included in the APP but associated with one another, or respectively belong to different APPs but be associated with one another. Embodiments are provided below for describing steps of the method in detail.

FIG. 2 is a flowchart illustrating a skin product fitting method according to an embodiment of the invention. Referring to both FIG. 1 and FIG. 2, the method of the present embodiment is applicable to the electronic apparatus 100 illustrated in FIG. 1, and detailed steps of the skin product fitting method of the present embodiment will be described below with reference to each device of the electronic apparatus 100 illustrated in FIG. 1.

First, the processor 120 executes the skin concern determining module 141 to capture a skin image of a current user by using the image capturing device 110 and analyze the skin image to determine a skin concern (step S202). In detail, in the present embodiment, skin care demands for normal people are divided in detail into a plurality of skin concerns, such as red spots, dark spots, dark circles, repair, moisturizing, anti-acne, anti-aging, anti-wrinkles, whitening, oil control, pore firmness, skin firmness, uneven skin tone and so on. Whenever a user uses the electronic apparatus 100, the electronic apparatus 100 determines a current skin condition of the user according to the currently obtained skin image of the user, determines a skin concern according to the current skin condition, which is regarded as a basis for subsequently providing product information fitting the current skin condition of the user.

Then, the processor 120 executes the skin type obtaining module 142 to obtain a skin type of the current user (step S204). The skin type obtaining module 142 obtains the skin type of the current user by, for example, querying the user or by detecting with a skin analyzer (not shown). The skin types include, for example, an oily type, a normal type, a dry type, a combination type and so on.

Lastly, the processor 120 executes the product information providing module 145 to sort a plurality of skin products stored in the product database at least according to the skin concern and the skin type so as to provide the product information fitting the skin condition of the current user (step S206). In detail, in the present embodiment, for example, a skin product database is established in the storage device 140 of the electronic apparatus 100, which includes a variety of skin products of different brands and for each skin product, the suitable skin type and corresponding skin concerns are marked according to the properties of the skin product. In other embodiments, the skin product database may also be established in a cloud apparatus and may be accessible by the electronic apparatus 100 to obtain desired product information.

By the method described above, whenever the user uses the electronic apparatus 100, the electronic apparatus 100 is capable of obtaining the skin concern and the skin type of the user for sorting the skin products in the skin product database so as to provide the product information most suitable for the current skin condition of the user.

For instance, if the skin concern of the current user is determined as moisturizing and the skin type of the user is determined as a dry type, the electronic apparatus may search moisturizing products suitable for dry skin from the skin product database, sort these products according to parameters, such as use effects (e.g., moisturizing degrees) and display the products column by column on the display for the user's reference in selecting skin products.

It should be mentioned that, in other embodiments, the invention further combines the skin concern and the skin type with information, including product preferences and a life profile of the user to select the products that better fit the current skin condition of the user. An embodiment is further provided below for detailed description.

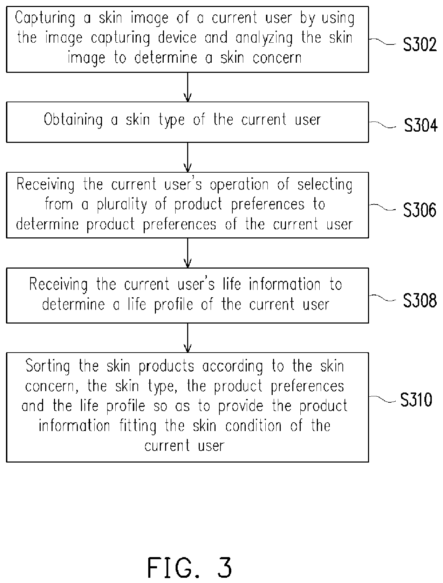

FIG. 3 is a flowchart illustrating a skin product fitting method according to an embodiment of the invention. Referring to both FIG. 1 and FIG. 3, the method of the present embodiment is applicable to the electronic apparatus 100 illustrated in FIG. 1, and detailed steps of the skin product fitting method of the present embodiment will be described below with reference to each device of the electronic apparatus 100 illustrated in FIG. 1.

First, the processor 120 executes the skin concern determining module 141 to capture a skin image of a current user by using the image capturing device 110 and analyze the skin image to determine a skin concern (step S302). In the present embodiment, the processor 120, for example, detects a skin condition of the user according to the skin image captured by the image capturing device 110 when the user first time uses the electronic apparatus 100, sets an expected skin goal suitable for the user's current skin condition according to a detection result and then, determines a skin concern suitable for the user's current skin condition according to skin concern determining processes developed for different expected skin goals.

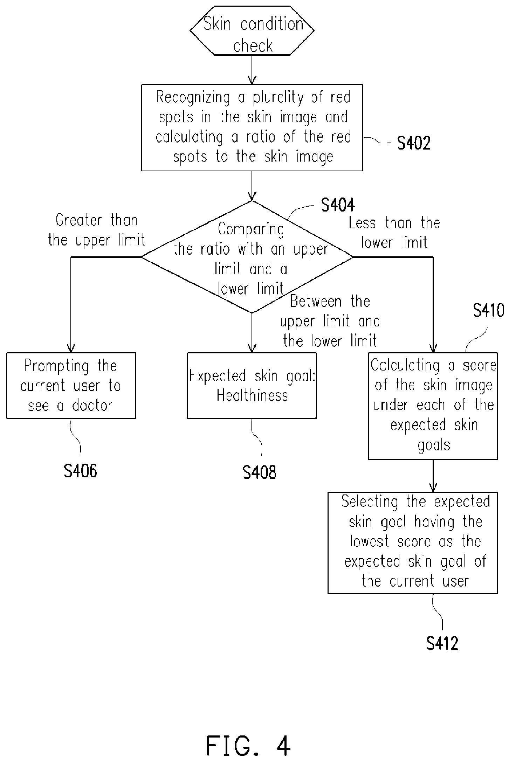

Specifically, FIG. 4 is a flowchart illustrating a method of detecting the skin condition according to an embodiment of the invention. Referring to both FIG. 1 and FIG. 4, the method of the present embodiment is applicable when the user first time uses the electronic apparatus 100. After the image capturing device 110 captures the user's skin image, the processor 120, for example, executes a skin condition detecting module (not shown) stored in the storage device 140 to recognize a plurality of red spots in the skin image and calculates a ratio of the red spots to the skin image (step S402). The aforementioned "red spots" refer to spot blocks in the skin image which are relatively red to the user's skin color and having a relatively large area and may probably appear in each part of the skin image. The skin condition detecting module may calculate a ratio of the red spots relative to an overall area of the face through, for example, a face image recognition technique.

Then, the skin condition detecting module compares the calculated ratio with an upper limit and a lower limit (step S404) so as to select one of a plurality of expected skin goals as an expected skin goal of the current user. Therein, the upper limit is, for example, a value between 15 and 25, and the lower limit is, for example, a value between 2 and 5, which are not limited herein. In addition, the expected skin goals include, for example, healthiness, clarity, firmness, texture and brightness.

If determining that the calculated ratio of the red spots is greater than the upper limit, thereby determining that the condition of the user's red spots is relatively serious, the skin condition detecting module prompts the current user to see a doctor by displaying a message on the display or other manners (step S406), without recommending the user to use any skin products to prevent the user's skin condition from getting worse.

If determining that the calculated ratio of the red spots is between the upper limit and the lower limit, thereby determining that the condition of the user's red spots is a moderate case, the skin condition detecting module may forcibly or preferentially select, for example, healthiness as the expected skin goal of the current user (step S408) to prevent the user from using relatively irritating skin products. It should be mentioned that in an embodiment, the skin condition detecting module may keep forcibly selecting healthiness as the expected skin goal of the current user, and the user is unable to change the expected skin goal before the ratio of the red spots of the user is less than the lower limit.

If determining that the calculated ratio of the red spots is less than the lower limit, thereby determining that the condition of the user's red spots is relatively minor, the skin condition detecting module may calculate a score of the skin image under each of a plurality of expected skin goals (step S410) and selects an expected skin goal having the lowest score (which refers to a target that relatively needs to be reinforced) as a default expected skin goal of the current user (step S412). Therein, "healthiness" of the skin may be calculated mainly by employing an amount and an area of red spots as main factors; "clarity" of the skin may be calculated according to an amount or an area of face spots (e.g., dark spots, red spots and redness) and a color darkness degree of the skin under the eyes; "firmness" of the skin may be calculated according to an area of fine lines and an amount and an area of pores; "texture" of the skin may be calculated according to an area of fine lines and wrinkles or an amount and an area of pores; and "brightness" of the skin may be calculated mainly by employing facial skin color uniformity as a main factor.

It should be mentioned that even though the skin condition detecting module sets the expected skin goal according to the user's initial skin condition when the user first time uses the electronic apparatus 100, the user may still be able to change the expected skin goal any time as desired in subsequent use so as to set the expected skin goal that meets the user's demand. Based on the expected skin goal set by the skin condition detecting module or the user, the skin concern determining module 141 determines the skin concern suitable for the user's the user's skin condition according to processes developed for different expected skin goals. Embodiments are provided below for detailed description.

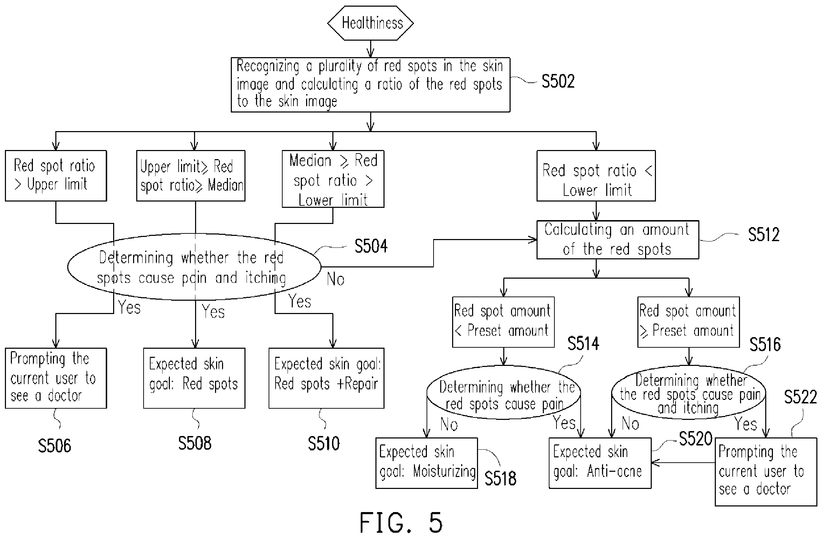

FIG. 5 illustrates a process of determining the skin concern when healthiness is selected as the expected skin goal according to an embodiment of the invention. Referring to FIG. 5, similar to the process of detecting the skin condition as illustrated in FIG. 4, the processor 120 first executes the skin condition detecting module to recognize a plurality of red spots in the skin image and calculate a ratio of the red spots to the skin image (step S502). Being different from the skin condition detecting process as described above, in the present embodiment, the processor 120 executes the skin concern determining module 141 to compare the ratio calculated by the skin condition detecting module with a preset upper limit, a preset lower limit and a preset median between the upper limit and the lower limit to determine the skin concern.

If determining that the ratio of the red spots is greater than the upper limit, between the upper limit and the median or between the median and the lower limit, the skin concern determining module 141 further determines whether these red spots cause pain and itching to the user (step S504). The skin concern determining module 141 queries the user by prompting a message, for example, and determines whether the red spots cause pain and itching to the user according to the user's answer provided by using the input device 130.

If the red spots cause pain and itching, the skin concern determining module 141 prompts the current user to see a doctor in a condition that the ratio of the red spots is greater than the upper limit (step S506), without recommending the user to use any skin products to prevent the user's skin condition form getting worse, determines "red spots" as the skin concern in a condition that the ratio of the red spots is less than or equal to the upper limit and greater than or equal to median (step S508), determines "red spots and repair" as the skin concern in a condition that the ratio of the red spots is less than the median and greater than or equal to the lower limit (step S510).

For the condition that the red spots do not cause pain and itching, or that the ratio of the red spots is less than the lower limit, the skin concern determining module 141 further calculates an amount of the red spots (step S512) and compares the amount with a preset amount. Therein, the preset amount is, for example, a value between 5 and 15, which is not limited herein. If the amount of the red spots is less than the preset amount, the skin concern determining module 141 queries the user whether the red spots cause pain (step S514) and determines whether the red spots cause pain according to the user's answer provided by using the input device 130. If the red spots cause pain, the skin concern determining module 141 determines "anti-acne" as the skin concern (step S518), and if not, the skin concern determining module 141 determines "moisturizing" as the skin concern (step S520).

On the other hand, if the amount of the red spots is greater than or equal to preset amount, the skin concern determining module 141 queries the user whether the red spots cause pain and itching (step S516) and determines whether the red spots cause pain and itching according to the user's answer provided by using the input device 130. If the red spots cause pain and itching, the skin concern determining module 141 first prompts the current user to see a doctor (step S522) and then, sets "anti-acne" as the skin concern (step S520), and if not, the skin concern determining module 141 directly determines "anti-acne" as the skin concern (step S520).

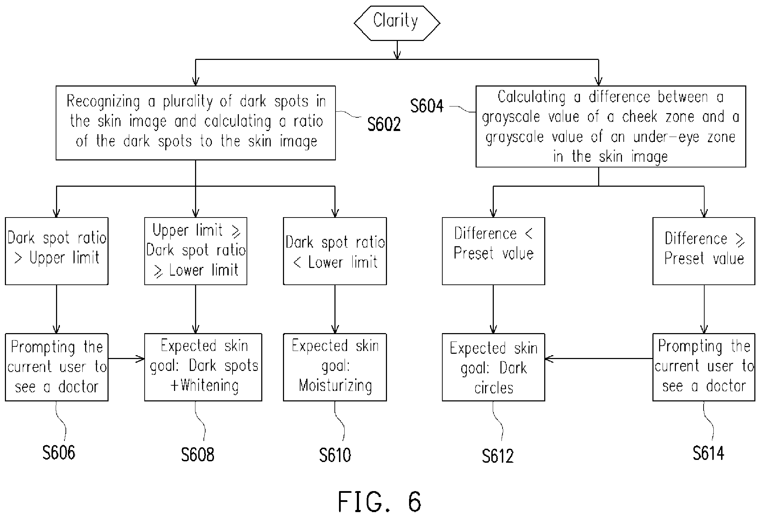

FIG. 6 illustrates a process of determining the skin concern when clarity is selected as the expected skin goal according to an embodiment of the invention. Referring to FIG. 6, the processor 120 first executes the skin concern determining module 141 and determines the skin concern respectively according to conditions of dark spots and dark circles.

In detail, for the condition of dark spots, the skin concern determining module 141 recognizes a plurality of dark spots in the skin image and calculates a ratio of these dark spots to the skin image (step S602) so as to compare the ratio with an upper limit and a lower limit. Therein, the upper limit is, for example, a value between 15 and 25, and the lower limit is, for example, a value between 1 and 3, which are not limited herein.

If the ratio of the dark spots is greater than the upper limit, the skin concern determining module 141 first prompts the current user to see a doctor (step S606) and then, sets "dark spots and whitening" as the skin concern (step S608), if the ratio of the dark spots is less than or equal to the upper limit, and greater than or equal to the lower limit, the skin concern determining module 141 directly determines "dark spots and whitening" as the skin concern (step S608), and if the ratio of the dark spots is less than the lower limit, the skin concern determining module 141 determines "moisturizing" as the skin concern (step S610).

On the other hand, for the condition of dark circles, the skin concern determining module 141 determines whether a difference between a grayscale value of a cheek zone and a grayscale value of an under-eye zone in the skin image is greater than or equal to a preset value (step S604). The preset value is, for example, a value between 2 and 5, which is not limited herein.

If the difference between the grayscale values is greater than or equal to the preset value, the skin concern determining module 141 first prompts the current user to see a doctor (step S614) and then, sets "dark circles" as the skin concern (step S612). If the difference between the grayscale values is less than the preset value, the skin concern determining module 141 directly determines "dark circles" as the skin concern (step S612).

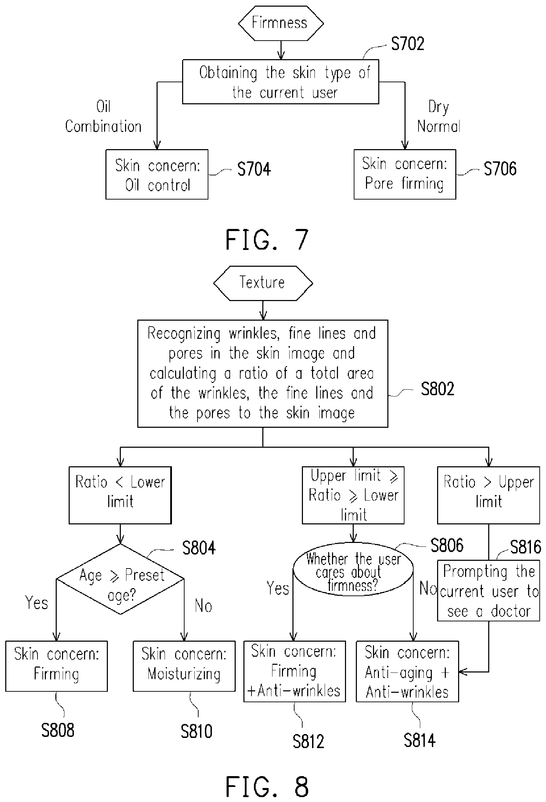

FIG. 7 illustrates a process of determining the skin concern when firmness is selected as the expected skin goal according to an embodiment of the invention. Referring to FIG. 7, the processor 120 first executes the skin concern determining module 141 to obtain a skin type of the current user (step S702). In an embodiment, the skin concern determining module 141, for example, displays skin type options, including an oily type, a dry type, a normal type and a combination type on the display of the electronic apparatus 100 and requests the user to select, thereby obtaining the skin type of the user according to the option selected by the user using the input device 130. In other embodiments, the skin concern determining module 141 may also detect the skin type of the current user by using a skin analyzer (not shown), which is not limited herein.

If the obtained skin type is the oily type or the combination type, the skin concern determining module 141 determines "oil control" as the skin concern (step S704), and if the obtained skin type is the dry type or the normal type, the skin concern determining module 141 determines "pore firming" as the skin concern (step S706).

FIG. 8 illustrates a process of determining the skin concern when texture is selected as the expected skin goal according to an embodiment of the invention. Referring to FIG. 8, the processor 120 first executes the skin concern determining module 141 to recognize wrinkles, fine lines and pores in the skin image, calculate a ratio of a total area of the wrinkles, the fine lines and the pores to the skin image (step S802), and compare the ratio with an upper limit and a lower limit. Therein, the upper limit is, for example, a value between 15 and 25, and the lower limit is, for example, a value between 2 and 5, which are not limited herein. It should be mentioned that the areas of the wrinkles, the fine lines and the pores may be obtained by, for example, multiplying respective areas of the wrinkles, the fine lines and the pores by a predetermined ratio (e.g., 25%, 25% or 50%) and summing up the products, which is not limited herein.

If the ratio is less than the lower limit, the skin concern determining module 141 further determines whether the current user's age is greater than or equal to a preset age (step S804). For example, the skin concern determining module 141 queries the user on the display of the electronic apparatus 100 whether the user's age is greater than the preset age and determines whether the user's age is greater than preset age according to the user's answer provided by using the input device 130. The preset age is, for example, a value between 25 and 40, which is not limited herein. If the user's age is greater than or equal to preset age, the skin concern determining module 141 determines "skin firmness" as the skin concern (step S808), and if the user's age is less than the preset age, the skin concern determining module 141 determines "moisturizing" as the skin concern (step S810).

On the other hand, if the ratio is less than or equal to the upper limit and greater than or equal to the lower limit, the skin concern determining module 141 further determines whether the current user cares about skin firmness (step S806). The skin concern determining module 141, for example, displays a message on the display of the electronic apparatus 100 to query the user whether he/she cares about skin firmness and confirm whether the user cares according to the option selected by the user by using the input device 130.

If the user cares about the skin firmness, the skin concern determining module 141 determines "skin firming and anti-wrinkles" as the skin concern (step S812), and if not, the skin concern determining module 141 determines "anti-aging and anti-wrinkles" as the skin concern (step S814).

Lastly, if the ratio is greater than the upper limit, the skin concern determining module 141 first prompts the current user to see a doctor (step S816) and then, sets "anti-aging and anti-wrinkles" as the skin concern (step S814).

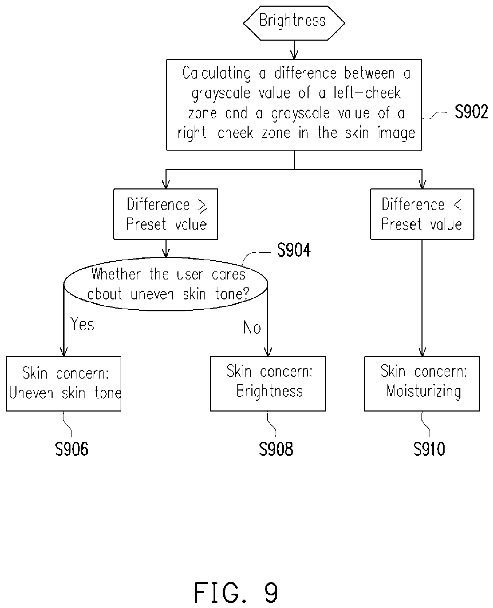

FIG. 9 illustrates a process of determining the skin concern when brightness is selected as the expected skin goal according to an embodiment of the invention. Referring to FIG. 9, the processor 120 first executes the skin concern determining module 141 to determine whether a difference between a grayscale value of a left-cheek zone and a grayscale value of a right-cheek zone in the skin image is greater than or equal to a preset value (step S902). Therein, the preset value is, for example, a value between 4 and 8, which is not limited herein.

If the difference between the grayscale values is greater than or equal to the preset value, the skin concern determining module 141 further determines whether the current user cares about partially uneven skin tone (step S904). The skin concern determining module 141, for example, displays a message on the display of the electronic apparatus 100 to query the user whether he/she cares about uneven skin tone to determine whether the user cares according to the option selected by the user by using input device 130.

If the user cares about the uneven skin tone, the skin concern determining module 141 determines "uneven skin tone" as the skin concern (step S906), and if not, the skin concern determining module 141 determines "whitening" as the skin concern (step S908).

On the other hand, if the difference between the grayscale values is less than the preset value, the skin concern determining module 141 directly determines "moisturizing" as the skin concern (step S910).

By the aforementioned stage by stage determination method, where the expected skin goal is first determined and the skin concern is determined according to different expected skin goals, accompanied with the manner of querying the user, the skin concern most suitable for the user's skin condition and demand may be obtained more accurately to serve as a basis for subsequently providing the product information.

Returning to the flow illustrated in FIG. 3, after the skin concern is determined, the electronic apparatus 100 executes the skin type obtaining module 142 by the processor 120 to obtain a skin type of the current user (step S304). The skin type obtaining module 142 obtains the skin type of the current user by, for example, querying the user or by using the skin analyzer, which is not limited herein.

It should be mentioned that being different from the above-described embodiment, in the present embodiment, the electronic apparatus 100 further obtains information, e.g., product preferences and a life profile in addition to the skin concern and the skin type to serve as the basis for subsequently providing the product information.

In detail, the processor 120 further executes the product preference determining module 143 to receive the current user's operation of selecting from a plurality of product preferences so as to determine product preferences of the current user (step S306). The product preference determining module 143, for example, queries the user about his/her product preference on the display of the electronic apparatus 100 and displays options of product preferences, such as fragrance-free, oil-free, alcohol-free, preservative-free (4-Hydroxybenzoate), sulfate-free and so on. The user may select one or multiple options by using the input device 130, and the selected product preferences may be employed as filter conditions in selecting the skin products, so as to select the products that satisfy the user's preferences (or filter out the products that the user dislikes).

Additionally, the processor 120 also executes the life profile determining module 144, thereby receiving the user's life information to determine a life profile of the current user (step S308). In detail, the life profile determining module 144 provides, for example, a plurality of options regarding the current user's living habits on the display of the electronic apparatus 100, where each of the options corresponds to at least one of a plurality of skin concerns. Then, the life profile determining module 144 receives the current user's operation of selecting from the options by using the input device 130, and calculates a number of times of each of the skin concerns being corresponded by the options selected by the current user as the life profile of the current user. The number of times of each skin concern is employed to weight the skin products meeting the skin concern when product information providing module 145 subsequently sorts the skin products.

For instance, Table 1 provided below lists options of the user's living habits and the corresponding skin concerns according to an embodiment of the invention.

**Table 1**

| | Option | Skin Concern |
|---|---|---|
| 1 | Do you smoke? | Pores /Anti-aging |
| 2 | Do you have poor sleep quality for a long term? | Anti-acne /Dark circles /Anti-aging /Whitening |
| 3 | Do you have high blood pressure? | Anti-acne /Dark circles |
| 4 | Do you have pimples before or after menstruation? | Anti-acne |
| 5 | Are you used to go to bed later than 1:00 at midnight? | Repair /Dark circles /Oil control /Anti-aging /Whitening |
| 6 | Do you have to work under the sun? | Whitening /Moisturizing /Red spots /Pores |

If the user simultaneously select options 2, 3 and 4, the skin concerns being corresponded and the numbers of times thereof are respectively anti-acne for 3 times, dark circles for twice, anti-aging for once and whitening for once, and the information is employed as the user's life profile so as to weight the skin products meeting the skin concerns to obtain the product information suitable for the user's life profile when the skin products are subsequently sorted.

Lastly, the processor 120 executes the product information providing module 145 to select and sort a plurality of skin products stored in the product database according to the skin concern, the skin type, the product preferences and the life profile, so as to provide the product information fitting the skin condition of the current user (step S310). To be specific, the product information providing module 145, for example, selects multiple skin products that simultaneously meet the skin concern, the skin type, the product preferences and the life profile from the skin products stored in the product database as a first group and sorts the selected skin products of the first group, selects multiple skin products that only meet the skin concern, the skin type and the product preference as a second group and sorts the selected skin products of the second group, selects multiple skin products that only meet the skin concern, the skin type and the life profile as a third group and sorts the selected skin products of the third group, and selects multiple skin products that only meet the skin concern and the skin type as a fourth group and sorts the selected skin products of the fourth group. Finally, the product information providing module 145 provides the sorted skin products of the first group, the second group, the third group and the fourth group in order as the product information fitting the skin condition of the current user.

In brief, a skin product that meets more conditions (i.e., the aforementioned skin concern, skin type, product preferences and life profile) may obtain a higher skinfit level and be displayed in the former among the recommended product information. In detail, Table 2 provided below lists skinfit levels and required conditions thereof according to an embodiment of the invention.

**Table 2**

| Skinfit Level | Required Condition | |
|---|---|---|
| High skinfit | • User's skin concern | |
| | • User's skin type | |
| | • User's product preferences | |
| | • User's life profile | |
| Moderate skinfit | A | • User's skin concern |
| | | • User's skin type |
| | | • User's product preferences |
| | B | • User's skin concern |
| | | • User's skin type |
| | | • User's life profile |
| Basic skinfit | • User's skin concern | |
| | • User's skin type | |

The aforementioned four conditions have to be met to achieve the high skinfit level, and the products meeting the four conditions are categorized into the first group; not only the skin concern and skin type have to be met but also the product preferences and the user's life profile have to be respectively met to achieve the moderate skinfit levels A and B, in which the product preferences are sorted prior to the life profile. The skin products meeting the moderate skinfit levels A and B are categorized into the second and the third groups. The conditions required for the basic skinfit level are the user's skin concern and skin type, and the skin products meeting the required conditions are categorized into the fourth group. Based on the categorization, the product information providing module 145, when providing the product information, also displays the corresponding skin products in an order of the first, the second, the third and the fourth groups. Namely, the skin product meeting the most required conditions is sorted on the top and preferentially provided for the user's reference.

For instance, Table 3 below lists the skinfit levels, the required conditions thereof and the sorting results according to an embodiment of the invention.

**Table 3**

| Skinfit Level | Required Condition | Sorting |
|---|---|---|
| High skinfit | • User's skin concern: dark circles | 1. Dark Circle + Dry + Oil-Free + Anti-acne |
| | • User's skin type: dry | 2. Dark Circle + Dry +Oil-Free + Pores |
| | • Product preferences: oil-free | |
| Moderate skinfit | | 1. Dark Circle + Dry + Oil-Free |
| | • User's life profile: anti-acne 2 for twice, and pores 1 for once | 1. Dark Circle + Dry + Anti-acne |
| | | 2. Dark Circle + Dry + Pores |
| Basic skinfit | | 1. Dark Circles + Dry |

In the sorting corresponding to the high skinfit level, since the number of times of anti-acne is greater than the number of times of pores in the user's life profile, a product for anti-acne is also sorted prior to a product for pores due to being weighted when the skin products are sorted. In the sorting corresponding to the moderate skinfit level, the product preferences (e.g., oil-free) are sorted prior to the user's life profile (e.g., anti-acne, and pores), and thus, a product with an oil-free property is sorted prior to the other products, while the product for anti-acne is also sorted prior to the product for pores among the other products. In the sorting corresponding to the basic skinfit level, products meeting the conditions regarding dark circles and dry type among the rest of the products are listed.

To summarize, in the skin product fitting method and the electronic apparatus therefor provided by the embodiments of the invention, the user's skin image is captured and analyzed to determine the expected skin goal fitting the user's current skin condition, and the skin concern is determined according to the processes developed for determining different expected skin goals. By combining the determined skin concern and the information (including the skin type, the product preferences and the life profile) obtained from the user, the skin products in the skin product database are grouped and sorted. Finally, the product information fitting the skin condition of the user is provided to the user as reference for selecting the skin products.

Although the invention has been described with reference to the above embodiments, it will be apparent to one of the ordinary skill in the art that modifications to the described embodiment may be made without departing from the spirit of the invention. Accordingly, the scope of the invention will be defined by the attached claims not by the above detailed descriptions.

## Claims

1. A skin product fitting method for an electronic apparatus (100) comprising an image capturing device (110), the method comprising:
capturing (S202) a skin image of a current user by using the image capturing device (110) and analyzing the skin image to determine a skin concern;
obtaining (S204) a skin type of the current user; and
sorting (S206) a plurality of skin products stored in a product database at least according to the skin concern and the skin type so as to provide product information fitting a skin condition of the current user.

2. The skin product fitting method according to claim 1, further comprising:
receiving (S306) the current user's operation of selecting from a plurality of product preferences to determine the product preferences of the current user;
receiving (S308) the current user's life information to determine a life profile of the current user; and
sorting (S310) the skin products according to the skin concern, the skin type, the product preferences and the life profile so as to provide the product information fitting the skin condition of the current user.

3. The skin product fitting method according to claim 2, wherein the step of sorting the skin products according to the skin concern, the skin type, the product preferences and the life profile so as to provide the product information fitting the skin condition of the current user comprises:
selecting a plurality of skin products that simultaneously meet the skin concern, the skin type, the product preferences and the life profile from the skin products as a first group and sorting the skin products in the first group;
selecting a plurality of skin products that only meet the skin concern, the skin type and the product preferences from the skin products as a second group and sorting the skin products in the second group;
selecting a plurality of skin products that only meet the skin concern, the skin type and the life profile from the skin products as a third group and sorting the skin products in the third group;
selecting a plurality of skin products that only meet the skin concern and the skin type from the skin products as a fourth group and sorting the skin products in the fourth group; and
providing the sorted skin products of the first group, the second group, the third group and the fourth group in order as the product information fitting the skin condition of the current user.

4. The skin product fitting method according to claim 2 or 3, wherein the step of receiving the current user's life information to determine the life profile of the current user comprises:
providing a plurality of options regarding the current user's living habits, wherein each of the options corresponds to at least one of a plurality of skin concerns; and
receiving the current user's operation of selecting from the options, calculating a number of times of each of the skin concerns being corresponded to by the options selected by the current user as the life profile, wherein the number of times of each skin concern is employed to weight the skin products meeting the skin concern when the skin products are sorted.

5. The skin product fitting method according to any one of claims 2 to 4, wherein the step of analyzing the skin image to determine the skin concern comprises:
recognizing a plurality of red spots in the skin image and calculating a ratio of the red spots to the skin image (S402);
comparing (S404) the ratio with an upper limit and a lower limit so as to select one of a plurality of expected skin goals as an expected skin goal of the current user, wherein the expected skin goals comprise healthiness, clarity, firmness, texture and brightness;
if the ratio is greater than the upper limit, prompting (S406) the current user to see a doctor;
if the ratio is between the upper limit and the lower limit, selecting (S408) the healthiness as the expected skin goal of the current user; and
if the ratio is less than the lower limit, calculating (S410) a score of the skin image under each of the expected skin goals and selecting the expected skin goal having the lowest score as the expected skin goal of the current user, wherein the step of analyzing the skin image to determine the skin concern when the healthiness is selected as the expected skin goal of the current user further comprises:
determining (S504) whether the red spots cause pain and itching;
if the red spots cause pain and itching, obtaining a median between the upper limit and the lower limit and comparing the ratio with the upper limit, the lower limit and the median;
if the ratio is between the upper limit and the median, determining (S508) the red spots as the skin concern; and
if the ratio is between the median and the lower limit, determining (S510) the red spots and repair as the skin concern.

6. The skin product fitting method according to any of the preceding claims, wherein before the step of analyzing the skin image to determine the skin concern, the method further comprises:
receiving the current user's operation of selecting from a plurality of expected skin goals and determining the skin concern according to the selected expected skin goal, wherein the expected skin goals comprise healthiness, clarity, firmness, texture and brightness.

7. The skin product fitting method according to claim 6, wherein when the firmness is selected as the expected skin goal of the current user, the step of analyzing the skin image to determine the skin concern comprises:
obtaining (S702) the skin type of the current user;
if the obtained skin type is an oily type or a combination type, determining (S704) oil control as the skin concern; and
if the obtained skin type is a dry type or a normal type, determining (S706) pore firming as the skin concern.

8. The skin product fitting method according to claim 6 or 7, wherein when the texture is selected as the expected skin goal of the current user, the step of analyzing the skin image to determine the skin concern comprises:
recognizing (S802) wrinkles, fine lines and pores in the skin image, calculating a ratio of a total area of the wrinkles, the fine lines and the pores to the skin image and comparing the ratio with an upper limit and a lower limit;
if the ratio is less than the lower limit, determining (S804) whether an age of the current user is greater than or equal to a preset age, determining skin firmness as the skin concern if the age is greater than or equal to the preset age and determining (S804) moisturizing as the skin concern if the age is less than the preset age;
if the ratio is between the upper limit and the lower limit, determining (S806) whether the current user cares about skin firmness, determining (S812) skin firming and anti-wrinkles as the skin concern if the current user does and determining (S814) anti-aging and anti-wrinkles as the skin concern if the current user does not; and
if the ratio is greater than the upper limit, determining (S814) anti-aging and anti-wrinkles as the skin concern.

9. The skin product fitting method according to any of claims 6 to 8, wherein when the brightness is selected as the expected skin goal of the current user, the step of analyzing the skin image to determine the skin concern comprises:
determining (S902) whether a difference between a grayscale value of a left-cheek zone and a grayscale value of a right-cheek zone in the skin image is greater than or equal to a preset value;
if the difference is greater than or equal to the preset value, determining (S904) whether the current user cares about partially uneven skin tone, determining (S906) uneven skin tone as the skin concern if the current user does and determining (S908) whitening as the skin concern if the current user does not; and
if the difference is less than the preset value, determining (S910) moisturizing as the skin concern.

10. An electronic apparatus (100), comprising:
an image capturing device (110), capturing a skin image of a current user;
a storage device (140), storing a plurality of modules; and
a processor (120), coupled to the image capturing device (110) and the storage device (140), accessing and executing the modules stored in the storage device (140), wherein the modules comprise:
a skin concern determining module (141), analyzing the skin image to determine a skin concern;
a skin type obtaining module (142), obtaining a skin type of the current user; and
a product information providing module (145), sorting a plurality of skin products stored in a product database at least according to the skin concern and the skin type so as to provide product information fitting a skin condition of the current user.

11. The electronic apparatus according to claim 10, wherein the modules further comprise:
a product preference determining module (143), receiving the current user's operation of selecting from a plurality of product preferences to determine the product preferences of the current user; and
a life profile determining module (144), receiving the current user's life information to determine a life profile of the current user, wherein
the product information providing module (145) sorts the skin products according to the skin concern, the skin type, the product preferences and the life profile so as to provide the product information fitting the skin condition of the current user.

12. The electronic apparatus according to claim 11, wherein the product information providing module (145) comprises:
selecting a plurality of skin products that simultaneously meet the skin concern, the skin type, the product preferences and the life profile from the skin products as a first group and sorting the skin products in the first group;
selecting a plurality of skin products that only meet the skin concern, the skin type and the product preferences from the skin products as a second group and sorting the skin products in the second group;
selecting a plurality of skin products that only meet the skin concern, the skin type and the life profile from the skin products as a third group and sorting the skin products in the third group;
selecting a plurality of skin products that only meet the skin concern and the skin type from the skin products as a fourth group and sorting the skin products in the fourth group; and
providing the sorted skin products of the first group, the second group, the third group and the fourth group in order as the product information fitting the skin condition of the current user.

13. The electronic apparatus according to claim 11 or 12, wherein the life profile determining module (144) comprises:
providing a plurality of options regarding the current user's living habits, wherein each of the options corresponds to at least one of a plurality of skin concerns; and
receiving the current user's operation of selecting from the options, calculating a number of times of each of the skin concerns being corresponded to by the options selected by the current user as the life profile, wherein the number of times of each skin concern is employed to weight the skin products meeting the skin concern when the skin products are sorted.

14. The electronic apparatus according to claim 12 or 13, wherein the skin concern determining module (141) further determines whether the red spots cause pain and itching, wherein
if the red spots cause pain and itching, a median between the upper limit and the lower limit is obtained, and the ratio is compared with the upper limit, the lower limit and the median;
if the ratio is between the upper limit and the median, the red spots are determined as the skin concern; and
if the ratio is between the median and the lower limit, the red spots and repair are determined as the skin concern.

15. The electronic apparatus according to any of claims 10 to 14, wherein the modules further comprise:
an expected skin goal selecting module, receiving the current user's operation of selecting from a plurality of expected skin goals and determining the skin concern according to the selected expected skin goal, wherein the expected skin goals comprise healthiness, clarity, firmness, texture and brightness.
